# EUROPEAN PATENT SPECIFICATION

(11) **EP 2 067 031 B1**
(45) Date of publication and mention of the grant of the patent: **27.07.2011**
(21) Application number: 07843378.6
(22) Date of filing: 27.09.2007
(51) Int. Cl.: G01N 33/00, A01K 67/00, C12N 15/00, C12N 15/63, A01N 63/00

(54) **RAPID IN VIVO MODEL FOR ANGIOGENESIS**
SCHNELLES IN-VIVO-MODELL FÜR ANGIOGENESE
MODÈLE IN VIVO RAPIDE DE L'ANGIOGENÈSE

(30) Priority: 27.09.2006 US 848001 P; 12.10.2006 US 851601 P
(43) Date of publication of application: 10.06.2009
(73) Proprietor: AntiCancer, Inc., San Diego, CA 92111 (US)
(72) Inventor: AMOH, Yasuyuki, San Diego, CA 92111 (US)
(74) Representative: Fisher, Adrian John
(86) International application number: PCT/US2007/079753
(87) International publication number: WO 2008/039932

(56) References cited:
- WO-A2-2005/042715
- WO-A2-2005/042715
- AMOH YASUYUKI ET AL: "Nestin-linked green fluorescent protein transgenic nude mouse for imaging human tumor angiogenesis" CANCER RESEARCH, vol. 65, no. 12, June 2005 (2005-06), pages 5352-5357, XP002539264 ISSN: 0008-5472
- JURJEES HASAN ET AL: "Quantitative Angiogenesis Assays in vivo - A Review" ANGIOGENESIS, KLUWER ACADEMIC PUBLISHERS, DO, vol. 7, no. 1, 1 March 2004 (2004-03-01), pages 1-16, XP019226873 ISSN: 1573-7209
- AKHTAR NASIM ET AL: "The sponge/Matrigel angiogenesis assay." ANGIOGENESIS, vol. 5, no. 1-2, 2002, pages 75-80, XP002539265 ISSN: 0969-6970
- AMOH YASUYUKI ET AL: "GFP-expressing vascularization of Gelfoam as a rapid in vivo assay of angiogenesis stimulators and inhibitors" BIOTECHNIQUES, vol. 42, no. 3, March 2007 (2007-03), pages 294-298, XP002539266 ISSN: 0736-6205
- AMOH Y. ET AL: 'Hair follicle-derived blood vascularize tumors in skin and are inhibited by Doxorubicin.' CANCER RES. vol. 15, 15 March 2005, pages 65 - 6
- HAYASHI K, ET AL.: 'Dual-color imaging of angiogenesis and its inhibition in bone and soft tissue sarcoma' J. SURG. RES. vol. 140, no. 2, 15 June 2007, pages 165 - 170, XP022083003 & DATABASE MEDLINE HAYASHI K. ET AL.: 'Dual-color imaging of angiogenesis and its inhibition in bone and soft tissue sarcoma'
- NORRBY K: 'In vivo models of angiogenesis' J. CELL MOL. MED. vol. 10, no. 3, July 2006 - September 2006, pages 588 - 612, XP008105503 & DATABASE MEDLINE [Online] NORRBY K. ET AL.: 'In vivo models of angiogenesis' Database accession no. (16989723)
- KIDNEY INT. vol. 71, no. 8, April 2007, pages 744 - 754, XP008105822 & DATABASE MEDLINE PATSCHAN D. ET AL.: 'Normal distribution and medullary-to-cortical shift of Nestin-expressing cells in acute renal ischemia'

## Description

### Technical Field

The invention relates to model systems for studying physiological phenomena and for determining the effectiveness of drugs and protocols designed to modulate them. In particular, it concerns an animal model that can be used to assess angiogenesis, and to assess the effect of various drugs on its development.

### Background Art

Angiogenesis, blood flow, intravascular tumor cell trafficking, and extravasation are critical steps in tumor growth, progression, and metastasis, and, therefore, are targets in worldwide drug discovery programs. The discovery and evaluation of anti-angiogenic substances have previously relied on *in vivo* methods such as a chorioallantoic membrane assay, a monkey iris neovascularization model, a disc angiogenesis assay, and various models using the cornea to assess blood vessel growth. These models have played an important role to understanding the mechanisms of blood vessel growth and its inhibition.

A novel transgenic nude mouse for the imaging of human tumor angiogenesis was developed in our laboratory. The stem-cell marker nestin is expressed in nascent blood vessels, and in this transgenic mouse, a regulatory element of nestin drives green fluorescent protein (ND-GFP) enabling nascent blood vessels to be visualized by their GFP expression. Many human and rodent cancer cell lines expressing red fluorescent protein (RFP) were implanted in the ND-GFP nude mice and grew extensively. ND-GFP was highly expressed in proliferating endothelial cells. Nascent blood vessels in the growing tumors were visualized by dual-color fluorescence imaging. Amoh, Y., et al., Cancer Res. (2005) 65:5352-5357. Doxorubicin was shown to inhibit nascent tumor angiogenesis as well as tumor growth in ND-GFP mice transplanted with the B16F10-RFP murine melanoma, Amoh, Y., *et al.* (*ibid*.) 2337-2343.

In addition, primary-tumor angiogenesis in ND-GFP transgenic nude mice with orthotopically transplanted MIA PaCa-2 human pancreatic cancer expressing RFP was visualized by dual-color imaging. Gemcitabine significantly decreased the mean nascent blood vessel density in the tumor as well as decreased tumor volume. These results demonstrated for the first time that gemcitabine is an inhibitor of angiogenesis as well as tumor growth in pancreatic cancer. Amoh, Y., et al., J. Surg. Res. (2006) 132:164-169. In further work, angiogenesis of liver metastasis of XPA-1-RFP human pancreatic cancer in ND-GFP transgenic nude mice was visualized by dual-color fluorescence imaging. ND-GFP was highly expressed in proliferating endothelial cells and nascent blood vessels in the growing liver metastasis. The density of nascent blood vessels in the liver metastasis was readily quantitated by ND-GFP expression. Gemcitabine significantly decreased the mean nascent blood vessel density in the liver metastases. Amoh, Y., et al., Anticancer Res. (in press).

A description of transgenic animals wherein fluorescent protein is expressed under control of a nestin-derived control system useful in observing neovasculature is also described in PCT publication WO 2005/042715 published 12 May 2005.

### Disclosure of Invention

The present invention provides an improvement in the angiogenesis detection system described above. In this method, angiogenesis can be measured independent of transplantation with a tumor by supplying appropriate growth factors that encourage neovascularization in a drug delivery matrix implanted subcutaneously in transgenic animals that produce fluorescent protein under control of a nestin-derived control sequence. The presence of new blood vessels in the growth matrix insert can then be observed directly by fluorescence microscopy. Control matrices lacking growth factor can be implanted in the same animal, and the effect of various drugs and protocols on the neovascularization can also be observed.

The present invention is advantageous as compared to tumor models of angiogenesis since the host animal need not be immunocompromised. As no xenogenic biological materials (other than the fluorescent protein per se) need be introduced, immunocompetent subjects may be used. By proper selection of the matrix, matrices that do not elicit immune responses can be employed.

Thus, in one aspect, the invention is directed to a transgenic non-human mammalian model for angiogenesis which model comprises a mammal modified to comprise a nucleotide sequence encoding a fluorescent protein under the control of a nestin control sequence so as to express a fluorescent protein in nascent blood vessel-forming cells and which further comprises one or more skin flaps containing a collagen gel block containing a growth factor for angiogenesis implanted in the subcutis, wherein said model is an in vivo non-tumor model, and is not immunocompromised.

Further embodiments are disclosed in the appended claims.

### Brief Description of Drawings

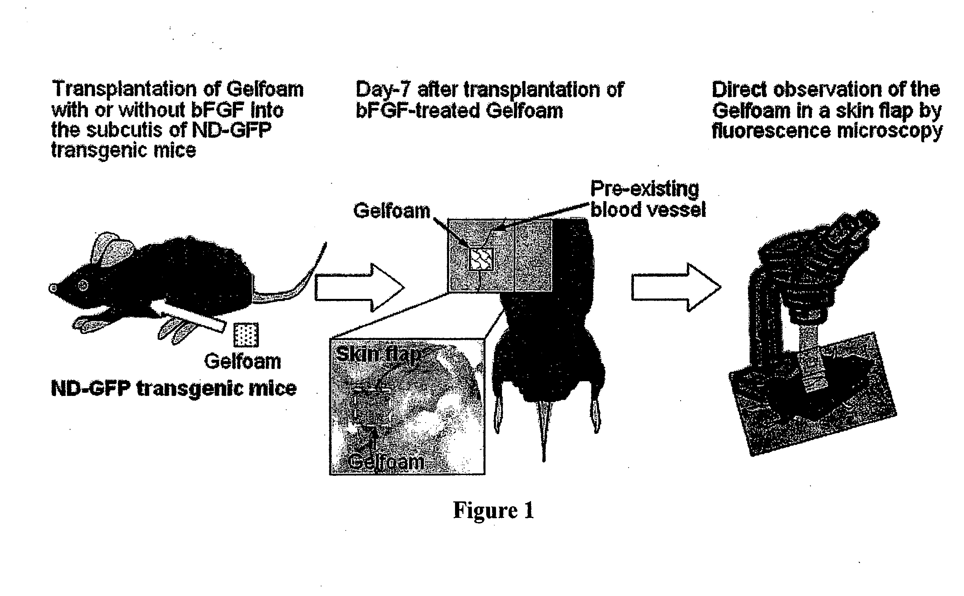

Figure 1 shows diagrammatically, one example of the overall process. As shown in the first part of the Figure, Gelfoam^{®} is transplanted in the subcutis into the flanks of mice modified to contain the nucleotide sequence for green fluorescent protein under control of the second intron enhancer of nestin (ND-GFP transgenic mice). Seven days after this transplantation, a skin flap is created containing the Gelfoam^{®} which has merged with a preexisting blood vessel. The Gelfoam^{®} in the skin flap is directly measured by fluorescence microscopy.

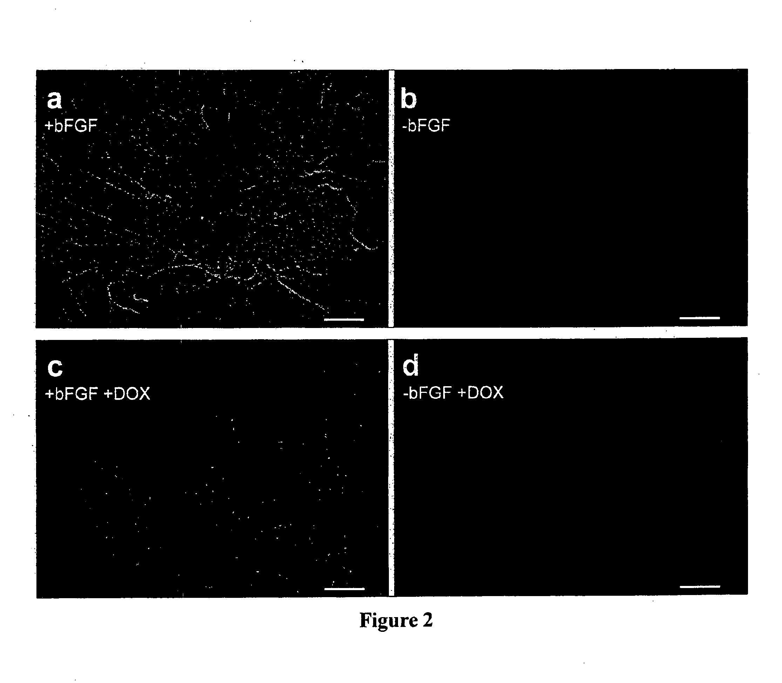

Figure 2 shows a comparison of angiogenesis observed under various conditions. Figure 2a shows angiogenesis in implanted Gelfoam^{®} when the Gelfoam^{®} contains basic fibroblast growth factor (bFGF). Figure 2b shows the lack of angiogenesis in a control Gelfoam^{®} without bFGF. Figure 2c shows the effect of doxorubicin on angiogenesis in Gelfoam^{®} containing bFGF. Figure 2d is an additional control showing lack of angiogenesis in controls that lack bFGF and are treated with doxorubicin.

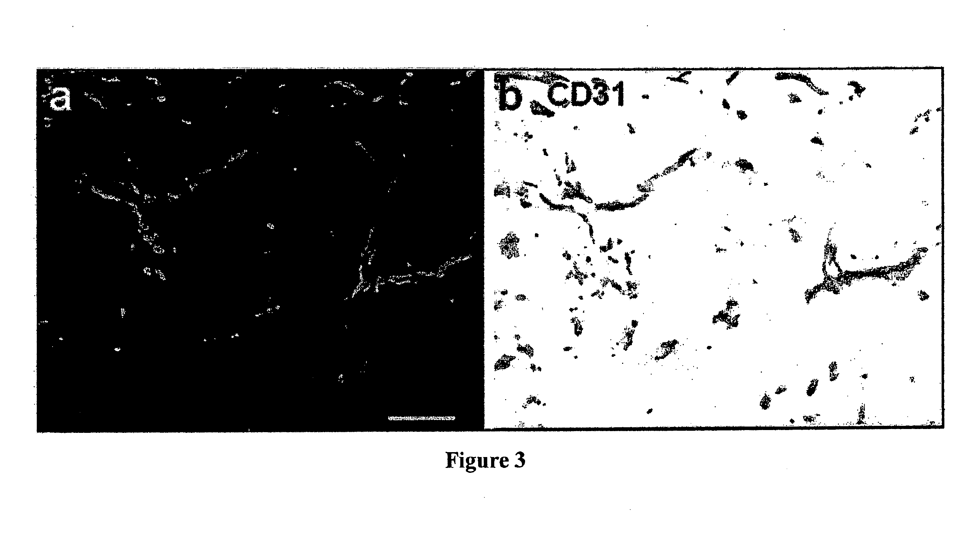

Figure 3 shows the correlation of fluorescence in histochemical sections of frozen vascularized Gelfoam^{®} with the endothelial cell marker CD31. Figure 3a shows the distribution of blood vessels as determined by fluorescence and Figure 3b shows the distribution of CD31.

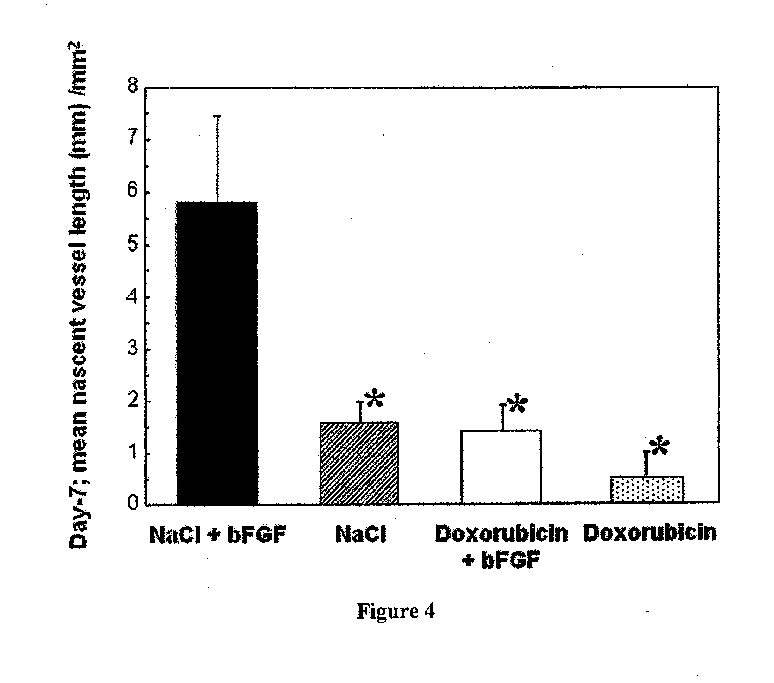

Figure 4 is a graph showing a comparison of the mean nascent vessel length in mm/mm² seven days after implantation of Gelfoam^{®} containing various components and with or without doxorubicin administration to the mouse.

### Best Modes for Carrying Out the Invention

The present invention provides a convenient and efficient model to measure the effect of any drug or protocol on the formation of neovasculature. Rather than relying on angiogenesis concomitant with tumor formation and growth, the model described herein cleanly induces angiogenesis in an artificial matrix and thus the effect of a drug or protocol specifically on angiogenesis can be determined and successful protocols and drugs identified. The model may be, but need not be, immunocompromised. It is a transgenic animal which produces a fluorescent protein in endothelial cells responsible for the formation of nascent blood vessels. Expression in these cells is accomplished by placing the nucleotide sequence encoding the fluorescent protein under a nestin control sequence. The transgenic animal, then, will selectively produce fluorescent protein in the appropriate blood vessel-forming cells. However, the specificity need not be complete, since the nascent blood vessels will be growing into a neutral matrix that does not offer background "noise."

The animal model is any mammal useful for this purpose such as a rodent, such as a rat or mouse, rabbits, primates, or other suitable subject.

The neovasculature will be encouraged to invade a synthetic matrix, such as Gelfoam^{®} (Upjohn). Gelfoam^{®} is illustrated herein, but other matrices such as Matrigel™ (BD Biosciences) could also be used. The matrix must simply be porous enough to support neovascularization and sufficiently retentive that it can supply growth factor locally in the subcutis. Various matrices are useful in the invention. The matrix serves both as a scaffolding for neovasculature and as a source of growth factors that are known to encourage angiogenesis. Typically, the animal model will have implanted both a matrix containing growth factors and a control matrix lacking such growth factors. In the control matrix, little, if any, neovasculature is typically observed. The size of the block of matrix to be implanted is dependent on the nature of the animal model. For mice, for example, typically, a sheet 2-6 mm on a side may be used. For large animals, larger sheets are employed. The thickness of the sheet is on the order of 1-3 mm. The sheet should be of such dimension that it can be included in a skin flap.

Suitable growth factors include angiogenin, angiopoietin 1, Del-1, fibroblast growth factors (acidic) (aFGF) and basic (bFGF)), follistatin, granulocyte colony stimulating factor (G-CSF), hepatocyte growth factor (HPGF) or scatter factor (SF), interleukin 8, leptin, placental growth factor, platelet derived endothelial growth factor (PDEGF), platelet derived growth factor BB (PDGF-BB), pleiotropin (PTN), proliferin, transforming growth factor-α (TGF-α), or β (TGF-β), tumor necrosis factor-a (TNF-a), vascular endothelial growth factor (VEGF), and progranulin. Some embodiments in particular use acidic or basic FGF, VEGF, and PDEGF.

With regard to labeling the endothelial cells, any fluorescent protein of sufficient brilliance can be used. A green fluorescent protein is illustrated herein, but other colors of fluorescence can also be employed, including red, yellow and blue. The fluorescent protein may also be modified to be compatible with the host, if necessary.

In a typical experiment, the animal is implanted subcutaneously in one flank with a small piece of the growth matrix that has been treated with growth factor, and in the other flank another piece of the matrix lacking growth factor. After a suitable time period, typically several days, but arbitrarily chosen as between four hours and two weeks in a typical embodiment, a skin flap containing the matrix is exposed and evaluated using a fluorescence microscope. The neovasculature is directly visible as fluorescent.

The effect of a drug or protocol can then be evaluated by comparing the angiogenesis observed in an animal administered the protocol as compared with a similarly constructed animal lacking the treatment. Any suitable method of administering drugs or providing protocols can be evaluated in this fashion.

Many previous models of angiogenesis are tumor models which require that the host of the tumor either be severely immunocompromised or syngeneic to the tumor. Because the synthetic growth matrix does not elicit an immune response, and the fluorescent protein either does not elicit such a response or can be modified so as to be immunotransparent, these restrictions are not required to use the present model. This permits both more realistic results and more convenient analysis. While nude mice are used in the example below, it is not necessary to use immunocompromised animals in the assay.

By "synthetic matrix" is meant either a matrix that is synthesized *de novo* from monomers or other components, or matrices that are isolated from living systems, but which are not tumors. Thus, for example, the exemplified Gelfoam is derived from pig gelatin.

The following example is offered to illustrate but not to limit the invention.

### Example 1

### Effect of Doxorubicin on Angiogenesis

Transgenic C57/B6 nude mice that produce green fluorescent protein (GFP) in endothelial cells were used. These transgenic mice (6-8 weeks old) were anesthetized with tribromoethanol. Gelfoam^{®} (Pharmacia & Upjohn company, Kalamazoo, MI) blocks (5 x 5 mm) were either treated with 300 ng basic fibroblast growth factor (bFGF) (Chemicon, Temecula, CA) in 75 µl RPMI 1640 medium (Cellgro, Herndon, VA) or not treated. The Gelfoam^{®} blocks were transplanted into the subcutis on both flanks of the transgenic mice, a treated block on one flank and an untreated block on the other. One group of mice was given daily ip injections of 5 µg/g of doxorubicin and another group was given 0.9% NaCl solution (vehicle control) at days 0, 1, and 2 after transplantation of Gelfoam^{®}. Skin flaps were made at day 7 under anesthesia. Angiogenesis was quantified by measuring the length of fluorescent nascent blood vessels in the skin flap by *in vivo* fluorescence microscopy imaging.

A Leica fluorescence stereo microscope model LZ12 equipped with a mercury lamp and a 50-W power supply was used. Selective excitation of GFP was produced through a D425/60 band-pass filter and a 470 DCXR dichroic mirror. Emitted fluorescence was collected through a long-pass filter (GG475; Chroma Technology, Brattleboro, VT) on a Hamamatsu C5810 3-chip cooled color charge-coupled device camera (Hamamatsu Photonics, Bridgewater, NJ). Images were processed for contrast and brightness and analyzed with the use of IMAGE PRO PLUS 3.1 software (Media Cybernetics, Silver Spring, MD). High-resolution images of 1024 x 724 pixels were captured directly on an IBM PC or continuously through video output on a high-resolution Sony VCR (model SLVR1000; Sony, Tokyo).

Co-localization of fluorescent neovasculature and CD31 in frozen sections of the vascularized Gelfoam^{®} was detected with the anti-rat immunoglobulin horseradish peroxidase detection kit (BD Pharmingen, San Diego, CA) following the manufacturer's instructions. The primary antibody was anti-CD31 mAb (1:50; Chemicon, Temecula, CA). Substrate-chromogen 3,3'-diaminobenzidine staining was used for antigen staining.

The experimental data are expressed as the mean ± SD. Statistical analysis was performed using the two-tailed Student's *t* test.

Figure 2 shows a comparison of the results. The bar in Figures 2a-2d represents a length of 500 µm. As shown in Figure 2a, a large complex of neovasculature is formed in the bFGF-containing Gelfoam^{®} patch in mice that were given control (0.9% NaCl) solution. Even in these mice, no significant neovasculature was observed in the Gelfoam^{®} that does not contain bFGF (Figure 2b). As shown in Figure 2c, mice administered doxorubicin showed greatly diminished neovasculature in the bFGF-treated foam, and no observable angiogenesis in the foam not containing bFGF.

Figure 4 is a graphical representation of the results shown in Figure 2. The mean nascent blood vessel length (in mm/mm²) is almost 6 in Gelfoam^{®} patches containing bFGF in mice administered sodium chloride as a control and some angiogenesis appears to be present in these mice even in Gelfoam^{®} not so treated. In mice administered doxorubicin, the extent of angiogenesis in the bFGF-treated Gelfoam^{®} is reduced below that of untreated Gelfoam^{®} in the sodium chloride treated mice.

Figure 3 shows correlation between the fluorescing blood vessels and the expression of CD31, a marker for endothelial cells. In Figure 3, the bar represents 100 µm. Frozen sections were made of the Gelfoam^{®} treated with bFGF at day 7. Figure 3a shows the distribution as determined by fluorescence of the new blood vessels and Figure 3b shows the distribution of CD31. The distribution of blood vessels is virtually identical to the distribution of CD31.

## Claims

1. A transgenic non-human mammalian model for angiogenesis which model comprises a mammal modified to comprise a nucleotide sequence encoding a fluorescent protein under the control of a nestin control sequence so as to express a fluorescent protein in nascent blood vessel-forming cells and which further comprises one or more skin flaps containing a collagen gel block containing a growth factor for angiogenesis implanted in the subcutis, wherein said model is an *in vivo* non-tumor model, and is not immunocompromised.

2. The transgenic non-human mammalian model of claim 1, which further comprises at least one skin flap containing a collagen gel block untreated with growth factor.

3. The transgenic non-human mammalian model of claim 1 or 2, wherein the growth factor is VEGF or bFGF.

4. A method to observe neovascularization in the transgenic non-human mammalian model for angiogenesis of any of claims 1-3, which method comprises directly observing by *in vivo* fluorescence microscopy the presence, absence or amount of fluorescence labeled nascent blood vessels formed by fluorescence labeled cells in said collagen gel block.

5. The method of claim 4, wherein the neovascularization is quantified by measuring the length of said nascent blood vessels.

6. The method of claim 4 or 5, which further comprises comparing the amount of nascent blood vessels in said collagen gel block comprising a growth factor with the amount of nascent blood vessels observed in a control not containing growth factor.

## Patentansprüche

1. Transgenes nicht-humanes Säugermodell für Angiogenese, wobei das Modell einen Säuger umfasst, der modifiziert ist, um eine Nukleotidsequenz zu umfassen, die ein fluoreszentes Protein unter der Steuerung einer Nestin-Steuerungssequenz codiert, so dass ein fluoreszentes Protein in naszenten Blutgefäß-bildenden Zellen exprimiert wird, und welches ferner einen oder mehrere, in die Subkutis implantierte Hautlappen umfasst, die einen Collagen-Gelblock enthalten, der einen Wachstumsfaktor für Angiogenese enthält, wobei das Modell ein *In-vivo*-Nicht-Tumor-Modell ist und nicht immunkompromittiert ist.

2. Transgenes nicht-humanes Säugermodell von Anspruch 1, welches ferner mindestens einen Hautlappen umfasst, der einen Collagen-Gelblock enthält, der nicht mit Wachstumsfaktor behandelt ist.

3. Transgenes nicht-humanes Säugermodell von Anspruch 1 oder 2, wobei der Wachstumsfaktor VEGF oder bFGF ist.

4. Verfahren zur Beobachtung der Neovaskularisierung in dem transgenen nicht-humanen Säugermodell für Angiogenese von beliebigen der Ansprüche 1-3, wobei das Verfahren das direkte Beobachten der Gegenwart, Abwesenheit oder Menge an Fluoreszenzmarkierten naszenten Blutgefäßen, die von Fluoreszenz-markierten Zellen in dem Collagen-Gelblock gebildet werden, durch *In-vivo-*Fluoreszenzmikroskopie umfasst.

5. Verfahren von Anspruch 4, wobei die Neovaskularisierung durch Messen der Länge der naszenten Blutgefäße quantifiziert wird.

6. Verfahren von Anspruch 4 oder 5, welches ferner das Vergleichen der Menge an naszenten Blutgefäßen in dem Collagen-Gelblock, der einen Wachstumsfaktor umfasst, mit der Menge an naszenten Blutgefäßen, beobachtet in einer Kontrolle, die keinen Wachstumsfaktor enthält, umfasst.

## Revendications

1. Modèle mammalien transgénique non humain pour l'angiogenèse, ledit modèle comprenant un mammifère modifié de manière à comprendre une séquence nucléotidique codant pour une protéine fluorescente sous le contrôle d'une séquence de contrôle de nestine de manière à exprimer une protéine fluorescente dans des cellules formant un vaisseau sanguin naissant et qui comprend en outre un ou plusieurs lambeaux de peau contenant un bloc de gel de collagène contenant un facteur de croissance pour l'angiogenèse implanté dans l'hypoderme, où ledit modèle est un modèle non tumoral *in vivo*, et n'est pas immunodéprimé.

2. Modèle mammalien transgénique non humain de la revendication 1, qui comprend en outre au moins un lambeau de peau contenant un bloc de gel de collagène non traité avec un facteur de croissance.

3. Modèle mammalien transgénique non humain de la revendication 1 ou 2, dans lequel le facteur de croissance est VEGF ou bFGF.

4. Procédé pour observer une néovascularisation dans le modèle mammalien transgénique non humain pour l'angiogenèse de l'une quelconque des revendications 1 à 3, ledit procédé comprenant l'observation directe par microscopie à fluorescence *in vivo* de la présence, l'absence ou la quantité de fluorescence de vaisseaux sanguins naissants formés par des cellules marquées par fluorescence dans ledit bloc de gel de collagène.

5. Procédé de la revendication 4, dans lequel la néovascularisation est quantifiée par mesure de la longueur desdits vaisseaux sanguins naissants.

6. Procédé de la revendication 4 ou 5, qui comprend en outre la comparaison de la quantité de vaisseaux sanguins naissants dans ledit bloc de gel de collagène comprenant un facteur de croissance avec la quantité de vaisseaux sanguins naissants observés dans un témoin ne contenant pas de facteur de croissance.
